# EUROPEAN PATENT APPLICATION

(11) **EP 2 623 606 A1**
(43) Date of publication of application: **07.08.2013**
(21) Application number: 11828406.6
(22) Date of filing: 27.09.2011
(51) Int. Cl.: C12P 7/06, C13K 1/02

(54) **METHOD FOR PRODUCING ETHANOL WITH CELLULOSIC BIOMASS AS STARTING MATERIAL**

(30) Priority: 30.09.2010 JP 2010221706
(71) Applicant: Kawasaki Jukogyo Kabushiki Kaisha, Kobe-shi, Hyogo 650-8670 (JP)
(72) Inventor: KUSUDA, Hiromasa, Hyogo 650-8670 (JP); IZUMI, Noriaki, Hyogo 650-8670 (JP); TAJIRI, Hironori, Hyogo 650-8670 (JP); TSUJITA, Shoji, Hyogo 650-8670 (JP); NISHINO, Takashi, Hyogo 650-8670 (JP)
(74) Representative: Matthews, Derek Peter
(86) International application number: PCT/JP2011/005419
(87) International publication number: WO 2012/042840

(57) **Abstract**

It is an object of the present invention to provide a method for producing ethanol by alcohol fermentation of a saccharide obtained by hydrolyzing cellulosic biomass in a supercritical or subcritical state, wherein excessive decomposition and caramelization of the saccharide are inhibited to prevent a reduction in yield of the saccharide, and flash steam is effectively used. The method for producing ethanol using cellulosic biomass as a raw material of the present invention is characterized in that in a saccharification/decomposition step, a slurry after saccharification/decomposition, which is taken from a hot water reaction vessel such as a pressure vessel, is flash-evaporated in a first flash tank so as to have a temperature of 150°C to 200°C (inclusive), and the retention time of the slurry after saccharification/decomposition is set at 3 minutes or less; the slurry after saccharification/decomposition, which is taken from the first flash tank, is further flash-evaporated in a second flash tank so as to have a temperature of 100°C to 120°C (inclusive); and first flash steam generated from the first flash tank is used as a heat source in the saccharification/decomposition step or a distillation step.

## Description

### [Technical Field]

The present invention relates to a method for producing ethanol (bioethanol) by alcohol fermentation of a saccharide after preparing the saccharide by hydrolyzing cellulosic biomass in a supercritical or a subcritical state.

### [Background Art]

As a part of utilization of biomass energy, there is an attempt to obtain ethanol by decomposing cellulose or hemicellulose that is a main component of the plant. In this case, the obtained ethanol is planned to be partly mixed principally in an automobile fuel or used as an alternative for gasoline for fuel use.

Main components of the plant include cellulose (polymer of glucose that is a C6 monosaccharide composed of 6 carbon atoms), hemicellulose (polymer of a C5 monosaccharide composed of 5 carbon atoms and C6 monosaccharide), lignin and starch, and ethanol is produced by a fermentation action of microorganisms such as yeast using, as a raw material, a saccharide such as a C5 monosaccharide, a C6 monosaccharide or an oligomer that is a complex thereof.

For decomposing cellulosic biomass such as cellulose or hemicellulose into a saccharide, three methods will be industrially used: 1) a method in which cellulosic biomass is hydrolyzed by oxidizing power of a strong acid such as sulfuric acid; 2) a method in which cellulosic biomass is decomposed by an enzyme; and 3) a method in which oxidizing power of supercritical water or subcritical water is used. However, for the acid decomposition method of 1), an added acid acts as an inhibitor to fermentation of yeast, a treatment is therefore essential for neutralizing the added acid before alcohol-fermenting a saccharide after decomposing cellulose or hemicellulose into the saccharide, and practical application may be difficult in terms of economy due to costs for the treatment. For the enzyme decomposition method of 2), it is possible to perform a treatment at an ordinary temperature and a constant pressure, but no effective enzyme has been found, production costs of the enzyme will be presumably increased if such an enzyme is found, and realization on the industrial scale is not in sight yet in terms of economy.

As the method of 3) in which cellulosic biomass is hydrolyzed by supercritical water or subcritical water to produce a saccharide, a method for producing a water-insoluble polysaccharide, wherein a cellulose powder is contacted with pressurized hot water at 240 to 340°C to be hydrolyzed, is disclosed in PTL 1. PTL 2 discloses a method, wherein finely divided biomass is hydrolyzed for a predetermined time period with hot water pressurized to a saturated water vapor pressure or higher at 140 to 230°C to decompose/extract hemicellulose, and thereafter hydrolyzed with pressurized hot water heated to a decomposition temperature of cellulose or higher to decompose/extract cellulose. PTL 3 discloses a method for producing glucose and/or a water-soluble cello-oligosaccharide, wherein cellulose having an average polymerization degree of 100 or more is contacted and reacted with supercritical water or subcritical water at a temperature of 250°C to 450°C (inclusive) and a pressure of 15 MPa to 450 MPa (inclusive) for 0.01 second to 5 seconds (inclusive), then cooled, and contacted with subcritical water at a temperature of 250°C to 350°C (inclusive) and a pressure of 15 MPa to 450 MPa (inclusive) for 1 second to 10 minutes (inclusive) to be hydrolyzed.

When cellulosic biomass is hydrolyzed by supercritical water or subcritical water, considerable energy is required for bringing a slurry of biomass into a high-temperature and high-pressure state. Thus, there are techniques for saving energy by recovering heat from a high-temperature and high-pressure slurry after hydrolysis. For example, PTL 4 discloses a technique, wherein a plurality of pressure vessels are sequentially operated, so that a saccharified solution slurry after hydrolysis is flash-evaporated to be cooled rapidly, and also flash steam is used for preheating a biomass slurry in another pressure vessel. PTL 4 discloses heat exchange performed between a saccharified solution slurry after flash evaporation and a biomass slurry charged into another pressure vessel. Similar techniques are also disclosed in PTLs. 5 and 6.

On the other hand, moisture or volatile substances in a slurry can be removed by flash evaporation. PTL 7 discloses an aqueous sewage sludge slurry is treated with hot water, and dewatered by multistage flash evaporation. PTL 8 discloses an apparatus, wherein when ground water or waste water such as industrial waste water contains a volatile organic compound such as carbon tetrachloride, trichloroethylene, tetrachloroethylene or benzene, the waste water is treated to remove the volatile organic compound therefrom. In this apparatus, a volatile organic compound is removed from waste water by multistage flash evaporation.

### [Citation List]

### [Patent Literature]

PTL 1: Japanese Laid-Open Patent Application Publication No. 2000-186102
PTL 2: Japanese Laid-Open Patent Application Publication No. 2002-59118
PTL 3: Japanese Laid-Open Patent Application Publication No. 2003-212888
PTL 4: International Publication No. WO 2008/050740
PTL 5: Japanese Laid-Open Patent Application Publication No. 2009-261275
PTL 6: Japanese Laid-Open Patent Application Publication No. 2009-261276
PTL 7: Japanese Laid-Open Patent Application Publication No. 6-39400
PTL 8: Japanese Laid-Open Patent Application Publication No. 2007-307466

### [Summary of Invention]

### [Technical Problem]

A method, in which cellulose and hemicellulose that are main constituents of biomass are saccharified and decomposed by high-temperature and high-pressure supercritical water or subcritical water, is a treatment method which is economical in terms of treatment costs and environmentally friendly because a treatment for neutralization of an acid is unnecessary in comparison with a hydrolysis method using a strong acid. However, the use of supercritical water or subcritical water has the disadvantage that due to its strong oxidizing power, decomposition of cellulose and hemicellulose is completed, and therefore a saccharide produced is excessively decomposed into an organic acid or the like unless cooling is performed immediately after completion of decomposition.

In a small decomposition apparatus on the laboratory scale, it may be possible to prevent excessive decomposition by rapidly cooling supercritical water or subcritical water in a heating vessel, but in a decomposition apparatus on the industrial scale, it is very difficult to rapidly cool a large amount of supercritical water or subcritical water in a short time. Thus, in a method for decomposing cellulosic biomass using high-temperature and high-pressure supercritical water or subcritical water, the yield of a saccharide is low on the plant scale, and this is one of causes for obstructing practical application.

In the methods for decomposing cellulosic biomass as disclosed in PTLs 4 to 6, rapid cooling is performed by flash-evaporating a saccharified solution slurry in a supercritical or subcritical state, but when the liquid amount of the saccharified solution slurry in a pressure vessel is too large at the time of flash evaporation, the saccharified solution slurry may bump into a flash steam pipe, causing solids in the slurry to stick in the pipe. If the slurry is gradually flash-evaporated for avoiding abrupt flash evaporation, such bumping can be prevented, but a saccharide contained in the saccharified solution slurry is excessively decomposed.

Even if the liquid amount of the saccharified solution slurry in a flash tank is moderate, the saccharified solution slurry after flash evaporation is required to be rapidly cooled to a subcritical or lower state in a shortest possible time from the viewpoint of preventing excessive decomposition of the saccharide. To do so, the retention time of the saccharified solution slurry in the pressure vessel, which is flash-evaporated, should be shortened wherever possible.

On the other hand, the techniques disclosed in PTLs 7 and 8 are intended to remove water or volatile organic substances from a slurry or waste water, and do not recover heat using flash steam. Also, it is not necessary that the storage time, in the tank, of the slurry or waste water after flash evaporation be shortened wherever possible.

It is an object of the present invention to provide a method for producing ethanol by alcohol fermentation of a saccharide obtained by hydrolyzing cellulosic biomass in a supercritical or subcritical state, wherein the method is capable of inhibiting excessive decomposition and caramelization of the saccharide to prevent a reduction in yield of the saccharide, and effectively using flash steam.

### [Solution to Problem]

The present inventors have continued vigorous studies for solving the above-mentioned problems. As a result, it has been found that by flash-evaporating a high-temperature and high-pressure saccharified solution slurry in two stages in two flash tanks after hydrolysis of cellulosic biomass, and shortening the storage time, excessive decomposition and caramelization of a saccharide can be prevented in the front-stage flash tank. Further, it has been found that flash steam discharged from the front-stage flash tank is hard to be decomposed, and are therefore easily used as a heat source in a saccharification/decomposition step or a heat source after alcohol fermentation, thus leading to completion of the present invention.

Specifically, the present invention relates to a method for producing ethanol using cellulosic biomass as a raw material, the method including:
a saccharification/decomposition step of performing saccharification/decomposition of cellulosic biomass by bringing a slurry of cellulosic biomass into a supercritical or subcritical state;
a fermentation step of alcohol-fermenting a saccharified solution obtained in the saccharification/decomposition step; and
a distillation step of distilling a fermented liquid obtained by the fermentation step to concentrate ethanol, wherein
in the saccharification/decomposition step, the slurry after saccharification/decomposition is flash-evaporated in a first flash tank so as to have a temperature of 150°C to 200°C (inclusive), and retention time, in the first flash tank, of the slurry after saccharification/decomposition is 3 minutes or less,
the slurry after saccharification/decomposition, which is taken from the first flash tank, is further flash-evaporated in a second flash tank so as to have a temperature of 100°C to 120°C (inclusive), and
a first flash steam generated from the first flash tank is used as a heat source in the saccharification/decomposition step or the distillation step.

After the saccharification/decomposition step, the high-temperature and high-pressure saccharified solution slurry taken from a hot water reaction vessel such as a pressure vessel is first flash-evaporated in the first flash tank so as to have a temperature of 150°C to 200°C (inclusive) (first flash evaporation), whereby the saccharified solution slurry can be prevented from bumping, causing solids to stick in a steam pipe. If the retention time of the slurry after saccharification/decomposition in the first flash tank is 3 minutes or less, and the saccharified solution slurry, the temperature and pressure of which are reduced, is transferred to the second flash tank as a second stage to flash-evaporate the slurry (second flash evaporation), excessive decomposition and caramelization of a saccharide can be suppressed. Since the flash steam discharged from the first flash tank has a temperature of 150°C to 200°C (inclusive) and is therefore hard to be condensed, it is easily transferred within the facility through the steam pipe and used as a heat source at the time of heating the saccharified solution slurry or a heat source at the time of distilling the fermented liquid obtained by alcohol fermentation of the saccharified solution slurry.

It is preferable that a part of the slurry after saccharification/decomposition, which is taken from the second flash tank, be continuously returned into the first flash tank. By this return, sticking of solids in the first flash tank and caramelization of the saccharide can be more effectively prevented.

It is also preferable that a part of the slurry after saccharification/decomposition, which is taken from the second flash tank, be intermittently returned into a pipe for transferring the saccharified solution slurry from the first flash tank to the second flash tank. By this return, sticking of solids in the pipe connecting the first flash tank and the second flash tank, and caramelization of the saccharide can be more effectively prevented.

It is also preferable that warm water be continuously injected into the first flash tank. By this injection, sticking of solids in the first flash tank and caramelization of the saccharide can be more effectively prevented as well.

It is also preferable that warm water be intermittently injected into the pipe for transferring the saccharified solution slurry from the first flash tank to the second flash tank. By this injection, sticking of solids in the pipe for transferring the saccharified solution slurry from the first flash tank to the second flash tank, and caramelization of the saccharide can be more effectively prevented.

The above-described object, other objects, features and advantages of the present invention will be apparent from detailed descriptions of the preferred embodiments below while referring to the attached drawings.

### [Advantageous Effects of Invention]

According to the present invention, energy can be saved for an overall plant facility by effectively using flash steam. At the same time, excessive decomposition and caramelization of a saccharide obtained from biomass can be prevented.

### [Brief Description of Drawings]

[Fig. 1] Fig. 1 shows a conceptual view for explaining Embodiment 1 of the present invention.
[Fig. 2] Fig. 2 shows a conceptual view for explaining Embodiment 2 of the present invention.
[Fig. 3] Fig. 3 shows a conceptual view for explaining Embodiment 3 of the present invention.
[Fig. 4] Fig. 4 shows a conceptual view for explaining one example of the conventional art.

### [Description of Embodiments]

Embodiments of the present invention will be described below with reference to drawings as appropriate.

### [Embodiment 1]

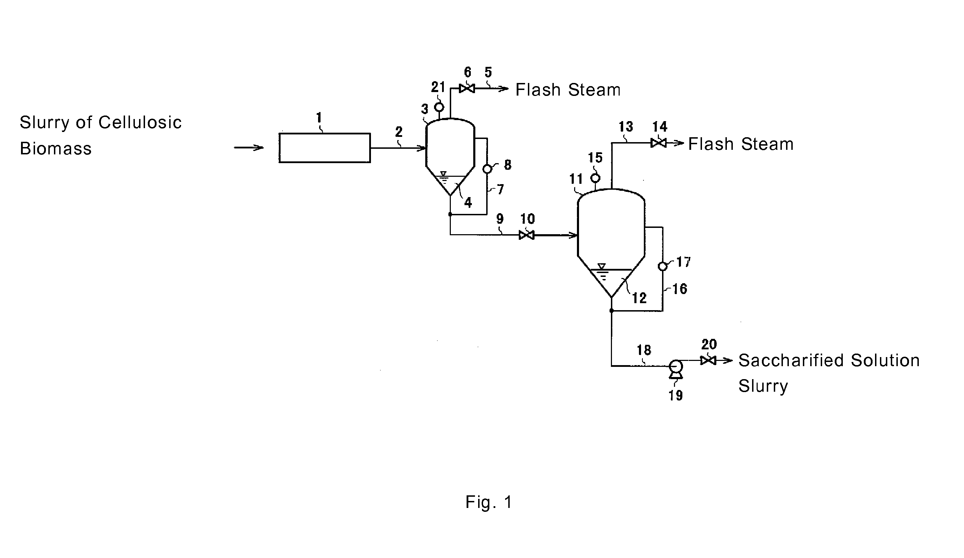

Fig. 1 is a conceptual view for explaining one embodiment of a method for saccharification/decomposition of cellulosic biomass according to the present invention. In the following embodiment, a method for saccharifying/decomposing cellulose.

### <1. Saccharification/decomposition step>

Cellulosic biomass (for example, vegetation biomass such as bagasse, beet sugar lees or straw) is ground to a grain size of several mm or less, and formed into a slurry having a solid concentration of about 5 to 50% by weight using water. This slurry is preheated as necessary, and thereafter fed into a hot water reaction vessel 1. The hot heat reaction vessel 1 serves the purpose as long as it is a reaction vessel that can heat and pressurize a slurry of cellulosic biomass into a supercritical or subcritical state, and may be a closed pressure vessel, or may be a heat exchanger such as a screw-type, spiral-type or shell-and-tube-type heat exchanger. The hot water reaction vessel 1 may be of a batch type or of a continuous type.

In the hot water reaction vessel 1, by keeping a slurry of cellulosic biomass at 3.3 MPa to 6.4 MPa and 240°C to 280°C for a fixed time period, cellulose in the slurry is hydrolyzed into a saccharide. A saccharified solution slurry after hydrolysis immediately passes through a passage 2 and is fed into a first flash tank 3.

In the first flash tank 3, the high-temperature and high-pressure saccharified solution slurry is flash-evaporated, and rapidly cooled to a temperature of 150°C to 200°C (inclusive) , which corresponds to a subcritical or lower state. If the slurry is decompressed in a stroke to 100°C or lower to be rapidly cooled, the saccharified solution slurry easily bumps. However, if the slurry is decompressed to 0.5 MPa to 1.6 MPa and thereby cooled to 150°C to 200°C (inclusive) on a temporary basis, excessive decomposition of the saccharide can be prevented while suppressing bumping of the saccharified solution slurry. The amount of a saccharified solution slurry 4 stored in the first flash tank 3 is preferably 1/3 of the internal volume of the first flash tank 3.

The aperture of a valve 6 provided in a steam pipe 5 is adjusted according to a measurement value from a pressure gauge 21, so that the pressure in the first flash tank 3 is adjusted to 0.5 MPa to 1.6 MPa. The first flash tank 3 is provided with a differential pressure type level meter 8 in a pipe 7 connecting the upper space of the first flash tank 3 and the saccharified solution slurry 4. The aperture of a valve 10 is adjusted according to a measurement value from the differential pressure type meter 8, so that the liquid level, i.e. the retention time, in the first flash tank 3 is adjusted. The saccharified solution slurry 4 cooled to 150°C to 200°C (inclusive) is transferred from a passage 9 to a second flash tank 11 so that the time for the slurry to be stored in the first flash tank 3 is 3 minutes or less because the saccharide may be caramelized if the slurry is left as it is.

In the second flash tank 11, the saccharified solution slurry 4 is flash-evaporated again, and cooled to 100°C to 120°C (inclusive). At this time, the pressure in the second flash tank 11 is 0.1 MPa to 0.2 MPa.

Flash steam generated in the second flash tank 11 is discharged into a steam pipe 13 connected to the ceiling surface of the second flash tank 11. The ceiling surface of the second flash tank 11 is provided with a pressure gauge 15, and the steam pipe 13 is provided with a valve 14. The aperture of a valve 14 is adjusted according to a measurement value from the pressure gauge 15, so that the pressure in the second flash tank 11 is adjusted to 0.1 MPa to 0.2 MPa.

A saccharified solution slurry 12 cooled to 100°C to 120°C (inclusive) is transferred from the second flash tank 11 to a passage 18. The passage 18 is provided with a pump 19, and the saccharified solution slurry 12 is submitted to a subsequent fermentation step. A pipe 16 connecting the upper space of the second flash tank 11 and the passage 18 is provided with a differential pressure type level meter 17. By adjusting the aperture of a valve 20 and the output of the pump 19 according to a measurement value from the differential pressure type level meter 17, the feed rate of the saccharified solution slurry 12 in the passage 18 is adjusted.

Since the saccharified solution slurry 12 in the second flash tank 11 is cooled to 100°C to 120°C (inclusive), excessive decomposition and caramelization of the saccharide do not occur. Therefore, the retention time of the saccharified solution slurry 12 in the second flash tank 11 is not limited.

### (Recovery of first flash steam)

Flash steam discharged from the first flash tank has a temperature of 150°C to 200°C, and is therefore hard to be condensed in the steam pipe. Flash steam is easily used at different locations in a facility as flash steam is transferred through the steam pipe. In a distillation step described later, ethanol is concentrated by distilling an alcohol-fermented liquid, but distillation of the fermented liquid in the distillation step consumes about 50 to 70% of the amount of steam used in an overall facility. Thus, if flash steam recovered from the first flash tank is transferred to a distillation device for carrying out the distillation step to recover heat, the amount of steam to be fed from a boiler can be reduced to save energy.

In a saccharification/decomposition step, considerable heat energy is required for bringing a slurry of cellulosic biomass into a supercritical or subcritical state. Thus, if flash steam recovered from the first flash tank is transferred to a heat exchanger for preheating a slurry, so that heat is recovered, the amount of steam for preheating a slurry can be reduced to save energy.

### (Recovery of second flash steam)

On the other hand, flash steam discharged from the second flash tank has a temperature of 100°C to 120°C, is therefore easily condensed and has a high wetness fraction. This flash steam is difficult to use as a utility in a facility, but can be used as, for example, a heat source for preheating a slurry of cellulosic biomass as a pretreatment in the saccharification/decomposition step.

### <2. Fermentation step>

The saccharified solution slurry obtained by the saccharification/decomposition step is converted into ethanol using yeast in a fermentation step. For the fermentation step, a known fermentation method can be employed.

### <3. Distillation step>

By the fermentation step, the saccharide contained in the saccharified solution slurry is converted into ethanol. Next, the alcohol-fermented liquid after the fermentation step is distilled to concentrate ethanol. A distilled liquid obtained by a distillation step is deprived of components other than solids and ethanol. For the distillation step, a distillation method known as a method for production of a distilled liquor can be employed.

### [Embodiment 2]

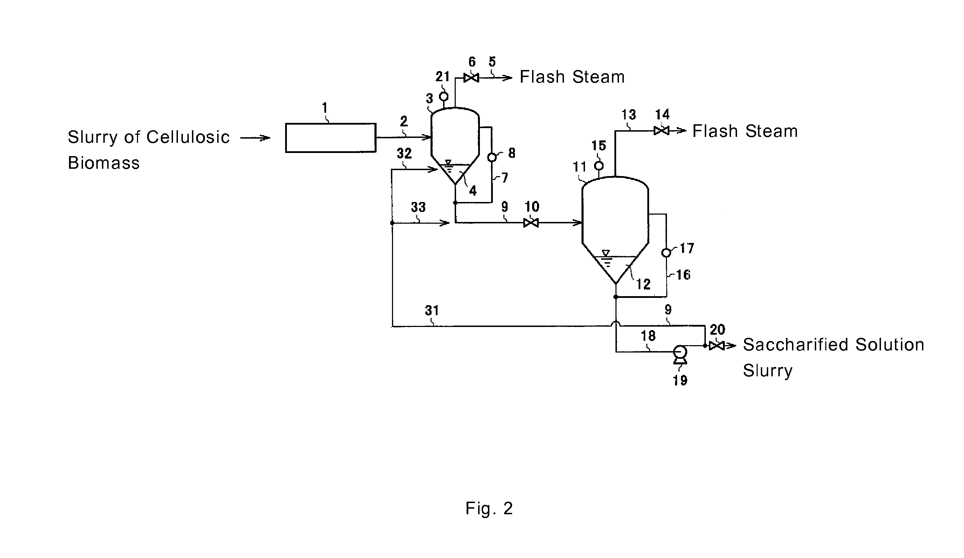

Fig. 2 is a conceptual view for explaining another embodiment of a method for saccharification/decomposition of cellulosic biomass according to the present invention. Here, only parts different from those of Embodiment 1 shown in Fig. 1 are described, and concerning common parts, descriptions are omitted.

In this embodiment, a part of a saccharified solution slurry transferred from a passage 18 to a fermentation step is returned to a first flash tank 3 and a passage 9 through return passages 31, 32 and 33. Any one of return passages 32 and 33 may be provided but more preferably, both thereof are provided.

When a saccharified solution slurry 4 is flash-evaporated in the first flash tank 3, the water content of the slurry decreases due to evaporation of water, so that solids easily stick inside the first flash tank and in the pipe of the passage 9. By continuously returning the saccharified solution slurry from the return passage 32 to the first flash tank 3, sticking of solids in the first flash tank 3 can be prevented, and the saccharified solution slurry 4 in the first flash tank 3 can be cooled to prevent caramelization of the saccharide as well. Similarly, by intermittently returning the saccharified solution slurry from the return passage 33 to the passage 9, sticking of solids in the pipe of the passage 9 can be prevented, and the saccharified solution slurry in the pipe of the passage 9 can be cooled to prevent caramelization of the saccharide as well.

The saccharified solution slurry is preferably gradually and continuously returned from the return passage 32 to the first flash tank 3. This is because the saccharified solution slurry 4 in the first flash tank 3 does not flow, and therefore easily stick inside the first flash tank 3.

On the other hand, it suffices that the saccharified solution slurry is intermittently returned from the return passage 33 to the passage 9. This is because in the passage 9, the saccharified solution slurry flows from the first flash tank 3 toward the second flash tank 11, and therefore solids are less likely to stick inside the first flash tank 3. The saccharified solution slurry may be returned from the return passage 33 at intervals of, for example, several hours.

### [Embodiment 3]

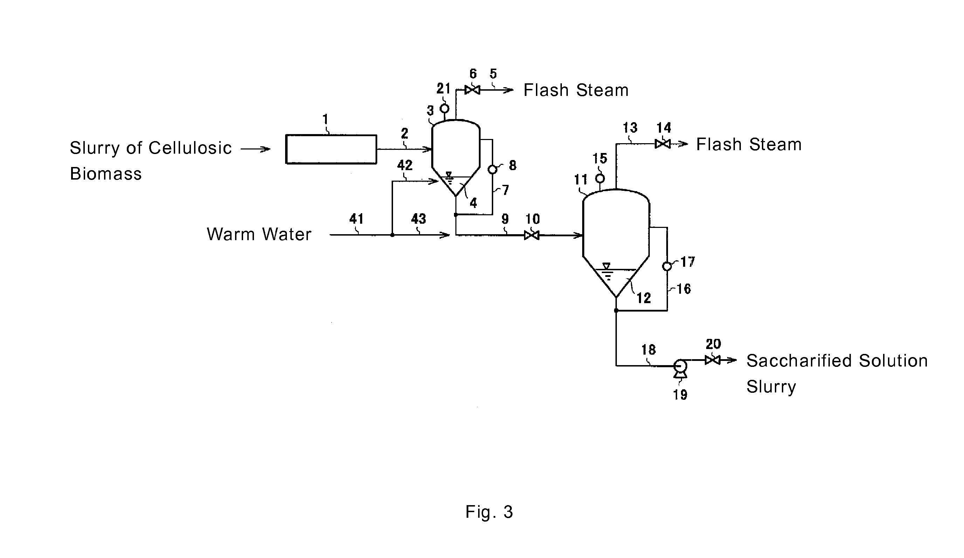

Fig. 3 is a conceptual view for explaining still another embodiment of a method for saccharification/decomposition of cellulosic biomass according to the present invention. Here, only parts different from those of Embodiment 1 shown in Fig. 1 are described, and concerning common parts, descriptions are omitted.

In this embodiment, warm water at about 80 to 200°C is injected into a first flash tank 3 and a passage 9 through warm water passages 41, 42 and 43. Any one of warm water passages 42 and 43 may be provided but more preferably, both thereof are provided.

As in Embodiment 2, warm water is preferably gradually and continuously injected from the warm water passage 42 into the first flash tank 3, and it suffices that warm water is intermittently injected from the warm water passage 43 into the passage 9. Unlike Embodiment 2, however, if the injected amount of warm water increases, the concentration of a saccharified solution slurry decreases, leading to a reduction in alcohol fermentation efficiency in a subsequent fermentation step. Thus, the injected amount of warm water should be smaller than the returned amount of the saccharified solution slurry.

### (Conventional art)

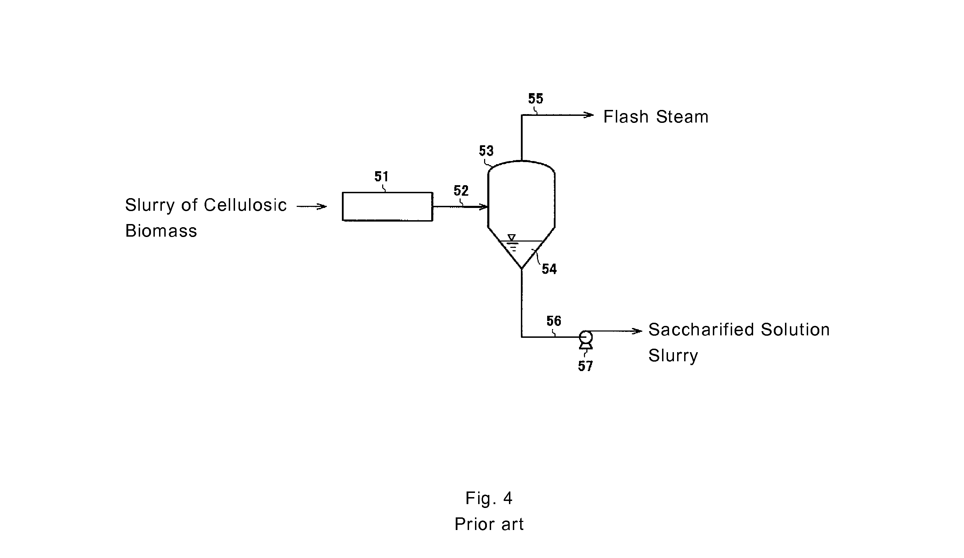

Fig. 4 is a conceptual view for explaining the conventional art of a method for saccharification/decomposition of cellulosic biomass. Cellulosic biomass is hydrolyzed by a hot water reaction vessel 51 as in Embodiments 1 to 3 of the present invention. A saccharified solution slurry passes through a passage 52 and is fed into a first flash tank 53. Thus, the saccharified solution slurry should be flash-evaporated to be rapidly cooled for preventing excessive decomposition of the saccharide.

At this time, a saccharified solution slurry 54 easily bumps because it should be flash-evaporated so as to make a transition from a supercritical state to 0.1 to 0.2 MPa and 100 to 120°C. When a part of the saccharified solution slurry 54 flows out into a steam pipe 55, solids stick therein. For removing solids, the steam pipe should be detached, so that operation efficiency is reduced, leading to an increase in production costs of ethanol.

It is preferable to shorten the retention time of the saccharified solution slurry 54 in the flash tank 53, but bumping of the saccharified solution slurry occurs if it is subjected to flash evaporation involving abrupt pressure reduction. On the other hand, if the saccharified solution slurry is gradually flash-evaporated for suppression of bumping, excessive decomposition and caramelization of the saccharide cannot be prevented, but there has not been such an idea that the storage time of the saccharified solution slurry in the flash tank is kept at a short time of 3 minutes or less.

From the above descriptions, many modifications or other embodiments of the present invention will be apparent to a person skilled in the art. Therefore, the above descriptions should be understood to be only illustrative, and are provided for the purpose of teaching a person skilled in the art the best aspects for implementation of the present invention. Details of the structure and/or function of the present invention can be substantially changed without departing from the spirit of the present invention.

### [Industrial Applicability]

The method for decomposing cellulosic biomass of the present invention is useful in the field of bioenergy as a method for producing a saccharide by decomposing cellulosic biomass.

### [Reference Signs List]

1
- 1:: hot water reaction vessel
- 2, 9, 18:: passage
- 3:: first flash tank
- 4, 12:: saccharified solution slurry
- 5, 13:: steam pipe
- 6, 10, 14, 20:: valve
- 7, 16:: pipe
- 8, 17:: differential pressure type level meter
- 11:: second flash tank
- 15, 21:: pressure gauge
- 19:: pump
- 31, 32, 33:: return passage
- 41, 42, 43:: warm water passage
- 51:: hot water reaction vessel
- 52:: passage
- 53:: flash tank
- 54:: saccharified solution slurry
- 55:: steam pipe
- 56:: passage
- 57:: pump

## Claims

1. A method for producing ethanol using cellulosic biomass as a raw material, the method comprising:
a saccharification/decomposition step of performing saccharification/decomposition of cellulosic biomass by bringing a slurry of cellulosic biomass into a supercritical or subcritical state;
a fermentation step of alcohol-fermenting a saccharified solution obtained in the saccharification/decomposition step; and
a distillation step of distilling a fermented liquid obtained by the fermentation step to concentrate ethanol, wherein
in the saccharification/decomposition step, the slurry after saccharification/decomposition is flash-evaporated in a first flash tank so as to have a temperature of 150°C to 200°C (inclusive), and retention time, in the first flash tank, of the slurry after saccharification/decomposition is 3 minutes or less,
the slurry after saccharification/decomposition, which is taken from the first flash tank, is further flash-evaporated in a second flash tank so as to have a temperature of 100°C to 120°C (inclusive), and
a first flash steam generated from the first flash tank is used as a heat source in the saccharification/decomposition step or the distillation step.

2. The method for producing ethanol according to claim 1, wherein a part of the slurry after saccharification/decomposition, which is taken from the second flash tank, is continuously returned into the first flash tank.

3. The method for producing ethanol according to claim 1, wherein a part of the slurry after saccharification/decomposition, which is taken from the second flash tank, is intermittently returned into a pipe for transferring the saccharified solution slurry from the first flash tank to the second flash tank.

4. The method for producing ethanol according to claim 1, wherein warm water is continuously injected into the first flash tank.

5. The method for producing ethanol according to claim 1, wherein warm water is intermittently injected into the pipe for transferring the saccharified solution slurry from the first flash tank to the second flash tank.
